# EUROPEAN PATENT SPECIFICATION

(11) **EP 2 417 199 B1**
(45) Date of publication and mention of the grant of the patent: **30.05.2018**
(21) Application number: 10713448.8
(22) Date of filing: 09.04.2010
(51) Int. Cl.: A61K 8/06, A61K 8/894, A61K 8/891, A61K 8/81, A61Q 19/08, A61Q 17/04, A61Q 1/10, A61Q 1/06, A61Q 1/02, A61K 8/31, A61Q 15/00, C08L 91/00, C08L 91/06

(54) **VOLATILE OILY COMPOSITION**
FLÜCHTIGE ÖLZUSAMMENSETZUNG
COMPOSITION HUILEUSE VOLATILE

(30) Priority: 10.04.2009 FR 0952400; 28.04.2009 US 173349 P
(43) Date of publication of application: 15.02.2012
(73) Proprietor: Biosynthis, 91410 Saint Cyr Sous Dourdan (FR)
(72) Inventor: BERNOUD, Thierry, F-91410 Saint Cyr Sous Dourdan (FR); RAMIANDRASOA, Parfait, 91120 Palaiseau (FR)
(74) Representative: Tripoz, Inès
(86) International application number: PCT/EP2010/054690
(87) International publication number: WO 2010/115973

(56) References cited:
- EP-A- 0 312 270
- EP-A- 2 014 274
- DE-U1-202005 011 257
- US-A1- 2004 241 200

## Description

The present invention relates to a volatile oily composition comprising, and preferably consisting of, a mixture of paraffins and optionally at least one non-volatile oil. It also relates to the cosmetic composition containing the abovementioned oily composition, and the cosmetic uses of said cosmetic composition, particularly for makeup and/or care for skin, lips, eyelashes and/or nails.

Although volatile silicones such as cyclomethicone have been considered for a long time to be harmless emollients and solvents for skin (see in particular International Journal of Toxicology, Vol. 10, No. 1, pp. 9-19, 1991), concerns have been raised in recent years in relation to the potential harmful effects thereof on the environment, or on human health (particularly in relation to octamethylcyclotetrasiloxane).

Research was thus undertaken to identify compounds suitable for offering a satisfactory alternative to volatile silicones, i.e. with similar volatility characteristics and similar behaviour in the formulation of cosmetic products, particularly in terms of the viscosity thereof, and having equivalent sensorial properties to those provided by volatile silicones, particularly in terms of applicability on the skin and the soft, "dry" and non-greasy texture of the film obtained.

In this way, the application US 2005/0079986 proposes an oily composition containing a mixture of a linear or branched dialkyl carbonate with an alkane, preferably branched and saturated, containing 8 to 40 carbon atoms. Similarly, the application US 2004/0241200 suggests the use of an association of isoparaffin (such as isododecane) with a neopentyl glycol polyester (such as neopentyl glycol diheptanoate) as a substitute for pentameric (D5) and tetrameric (D4) cyclomethicones. This mixture is particularly marketed by INOLEX under the brand name Lexfeel® D4 and D5. PRESPERSE LLC also offers, under the brand name SiClone® SR-5, a mixture of C₁₃-C₁₆ and C₁₂-C₁₄ isoparaffins with C₁₃-C₁₅ alkanes, as a cyclomethicone substitute. Finally, other attempts consisted of offering raw materials from renewable sources. This is the case of caprylyl isostearate which is the result of the esterification of 2-octanol by a mixture C₁₈ fatty acid isomers (US-6,126,951), but also of the mixture of linear paraffins disclosed in WO 2008/155059. This mixture comprises at least two hydrocarbons which differ from at least two carbon atoms. These hydrocarbons are preferably chosen from those having an odd number of carbon atoms from C₁₁ to C₂₁, most preferably a mixture of C₁₁ and C₁₃ hydrocarbons. Other linear paraffins are those marketed by SASOL under the trade name Linpar® 10-13, which comprise a mixture of linear hydrocarbons from C₉ to C₁₄ and more, and by SONNEBORN under the trade name Lilac®, which comprise a mixture of alkanes from C₁₄ to C₂₂.

However, there is still a need for an oily composition having very similar physicochemical and sensorial characteristics to those of cyclomethicones and consequently capable of being substituted for same in the formulation, in particular, of cosmetic products.

Very unexpectedly, the Applicant discovered that this need was met by an oily composition based on paraffins which, unlike the substitutes of the prior art, such as isoparaffins and fatty acid esters, have a linear and non-branched structure.

The present invention thus relates to a volatile oily composition comprising:
(a) from 50 to 100% by weight of a mixture of linear paraffins consisting of:
   (i) 70 to 99% by weight of at least one linear paraffin selected from C₈, C₁₀, C₁₂ paraffins and mixtures thereof,
   (ii) 1 to 30% by weight of at least one C₁₄ to C₂₄ linear paraffin, and
(b) from 0 to 50% by weight of at least one non-volatile oil selected from: mineral or synthetic branched hydrocarbons, (poly)esters and (poly)ethers, C₆-C₂₀ fatty acid triglycerides, vegetable oils, dialkyl carbonates, branched and/or unsaturated fatty acids, branched and/or unsaturated fatty alcohols, silicon oils, fluorosilicone oils, fluorinated oils, and mixtures thereof, wherein the volatile oily composition has a flash point measured according to the ASTM D93 standard below 100°C, and a vapour pressure in the region of 0.13 Pa (0.001 mm Hg) to 3.99.10⁴Pa (300 mm Hg) at ambient temperature (20°C) and atmospheric pressure, and wherein the non-volatile oil exhibits a vapour pressure less than 0.13 Pa (0.001 mm Hg) at room temperature and atmospheric pressure.
Firstly, it should be noted that, in the description and the following claims, the expression "between" should be understood as including the cited limits.

Indeed, the Applicant demonstrated that the abovementioned association of raw materials had a volatility within the same range as cyclomethicones, i.e. a vapour pressure in the region of 0.13 Pa (0.001 mm Hg) to 3.99.10⁴ Pa (300 mm Hg) and preferably from 1.33 Pa (0.01 mm Hg) to 1.33.10³ Pa (10 mm Hg) at ambient temperature (20°C) and atmospheric pressure, and an emollient nature and easy application on the skin, such that compositions containing this association leave, on the skin, after penetration in the outer layers thereof, a film having a soft, non-greasy touch and a reduced gloss similar to those of cyclomethicones. This oily composition has a flash point (measured as per the ASTM D93 standard) below 100°C, preferably between 50 and 95°C, for example between 70 and 90°C, more preferably between 75 and 85°C and, even better, between 75 and 80°C, and/or a kinematic viscosity below 5 mm².s⁻¹ (cSt) or between 1 and 3 mm².s⁻¹ (cSt), at 40°C.

This association of raw materials may thus partly or completely substitute the volatile silicones conventionally used in cosmetic compositions and more particularly cyclopentasiloxane and mixtures containing same (such as DC345® sold by DOW CORNING).

The paraffins (or fatty alkanes) contained in the oily composition according to the invention may be advantageously obtained according to a method comprising the following successive steps:
1- dehydration of at least one C₈-C₂₄ fatty alcohol to obtain an alkene, and
2- hydrogenation of said alkene to an alkane.

The first step of this method may particularly be implemented as described in the document US 2008/0287722, i.e. at a temperature of 190 to 260°C, preferably of 220 to 250°C, in the presence of a dehydration catalyst consisting of trifluoromethane sulphonic acid, which may represent 0.5 to 3% of the weight of the alcohol, for example. The alcohol dehydrated in this step may be obtained from plant sources and particularly be obtained by saponification of natural oils or fats. However, it is preferable for it to be obtained according to a method comprising a transesterification step of fatty acid triglycerides, preferably of plant origin, followed by a hydrogenation step of the fatty acid esters (for example methyl esters) obtained. The use of fatty alcohols of plant origin leads to alkenes containing an even number of carbon atoms, generally in mixture form. This mixture may also comprise a minor amount of branched alkenes.

The second step of this method may be implemented in a conventional manner for those skilled in the art, according to techniques used in the food processing industry for hydrogenating oils, and particularly placing the alkene(s) in contact with a catalyst comprising a transition metal. The alkanes obtained preferably contain, as above, an even number of carbon atoms. They are linear, although they may also include a minor amount of branched alkanes.

Obviously, this method may further comprise other steps (preliminary, intermediate and/or subsequent steps) than those mentioned above.

Alternatively, commercially available paraffins, such as those available from SASOL under the brand name Parafol® (particularly Parafol® 14-97 for tetradecane) may be used in the oily composition according to the invention.

The oily composition according to the invention comprises 70 to 99% by weight, for example 70 to 90% or 90 to 99% by weight, C₈, C₁₀ and/or C₁₂ paraffin(s) and 1 to 30% by weight, for example 10 to 30% or 1 to 10% by weight, C₁₄ to C₂₄ paraffin(s). These two types of paraffins may be obtained separately and mixed, or obtained jointly from a mixture of fatty alcohols, particularly of plant origin, according to the method described above. Among the C₈, C₁₀ and/or C₁₂ paraffin(s), the C₁₂ paraffin is preferred. Moreover, as explained above, the C₁₄ to C₂₄ paraffin(s) are preferably chosen among those having an even number of carbon atoms, still preferably the C₁₄ paraffin.

Furthermore, preferably, the oily composition according to the invention comprises, or consists only of, dodecane and tetradecane.

In addition to the abovementioned paraffins, and according to the desired volatility, the oily composition according to the invention may contain at least one non-volatile oil. According to the present invention, the term "oil" refers to a liquid compound at ambient temperature (25°C), which, when introduced at a rate of at least 1% by weight in water at 25°C, is not at all soluble in water, or soluble at a rate of less than 10% by weight, with reference to the weight of oil introduced into the water. In this description, the term "non-volatile oil" refers to an oil remaining on the skin at ambient temperature and atmospheric pressure for a plurality of hours, in the absence of friction, and/or having a vapour pressure less than 0.001 mm Hg under these conditions.

Examples of non-volatile oils include: mineral or synthetic branched hydrocarbons, synthetic (poly)esters and (poly)ethers and particularly (poly)esters of C₂-C₂₄ (preferably C₆-C₂₀) acids and C₂-C₂₄ (preferably C₆-C₂₀) alcohols or polyols, which are advantageously branched, C₆-C₂₀ fatty acid triglycerides, vegetable oils, dialkyl carbonates such as dicaprylyl carbonate, branched and/or unsaturated fatty acids (such as linoleic and linolenic acids), branched and/or unsaturated fatty alcohols (such as octyldodecanol or hexyldecanol), silicone oils, fluorosilicone oils, fluorinated oils, and mixtures thereof.

The term "hydrocarbon" refers to an oil containing only hydrogen and carbon atoms. Examples of non-volatile hydrocarbon oils are polybutene, hydrogenated polyisobutene, polydecene, hydrogenated polydecene, squalane, non-volatile paraffin oils and mixtures thereof.

The (poly)esters of C₂-C₂₄ acids and C₆-C₂₀ alcohols and polyols, which represent the preferred category of non-volatile oils according to the invention, particularly include mono- and diesters such as ethyl acetate, isopropyl acetate, oleyl acetate, isononyl isononanoate, ethylhexyl isononanoate, hexyl neopentanoate, ethylhexyl neopentanoate, isodecyl neopentanoate, isostearyl neopentanoate, heptyl undecylenate, neopentylglycol diheptanoate, neopentylglycol diethylhexanoate, pentaerythrityl tetraethylhexanoate, propanediol dicaprylate, neopentylglycol dicaprylate / dicaprate, isopropyl myristate, isopropyl palmitate, hexyl laurate, the mixture of coco caprate and caprylate, C₁₂ to C₁₅ alcohol benzoates, and mixtures thereof.

Examples of vegetable oils are in particular wheat germ, sunflower, grape seed, sesame, corn, apricot, castor, shea, avocado, olive, soya, sweet almond, palm, rapeseed, cotton, hazelnut, macadamia, jojoba, alfalfa, poppy seed, pumpkin seed, sesame, marrow, rapeseed, blackcurrant, evening primrose, millet, barley, quinoa, rye, safflower, candlenut, passiflora, musk rose or camellia oils.

The term "silicone oil" refers to an oil comprising at least one silicon atom, and particularly at least one Si-O group. Non-volatile silicon oils particularly include polydimethylsiloxanes containing at least 8 silicon atoms, polyalkylmethylsiloxanes in which the alkyl chain contains 8 to 20 carbon atoms and oils identified using the INCI name phenyl trimethicone.

The oily composition according to the invention contains 50 to 100% by weight of paraffin mixture and 0 to 50% by weight of non-volatile oil(s), for example 70 to 95% by weight, preferably 85 to 95% by weight, of paraffin mixture and 5 to 30% by weight, preferably 5 to 15% by weight, of non-volatile oil(s).

Oily compositions according to this invention are also marketed by BIOSYNTHIS under the trade name Vegelight®.

The oily composition described above is advantageously intended for use in the formulation of a cosmetic composition, particularly suitable for making up and/or caring for and/or protecting skin, lips, eyelashes and/or nails.

Therefore, the present invention also relates to a cosmetic composition containing the oily composition described above, along with the uses thereof.

This cosmetic composition comprises a physiological acceptable and preferably cosmetically acceptable medium, i.e. which has no harmful side effects and particularly which does not cause unacceptable redness, inflammation, tautness or smarting for a user of cosmetic products.

This medium optionally comprises water and/or at least one oil, in addition to the abovementioned oily composition. The cosmetic composition according to the invention may thus be an anhydrous composition, an emulsion such as a water-in-oil (W/O) emulsion, an oil-in-water (O/W) emulsion or a multiple emulsion (particularly W/O/W or O/W/O), or a dispersion.

The oils particularly include those mentioned above as constituent (b) of the oily composition according to the invention, and volatile oils, which may particularly be selected from: branched hydrocarbons such as isododecane or isohexadecane, linear volatile silicone oils such as hexamethyldisiloxane or octamethyl trisiloxane, volatile fluorinated oils such as nonafluoromethoxybutane, and mixtures thereof.

The cosmetic composition according to the invention may further contain at least one of the following constituents: a fatty phase gelling or structuring agent, particularly a wax, a gum, an olefin copolymer or a silicone elastomer; a binder, particularly a fatty acid soap; an aqueous phase gelling or thickening agent, such as an acrylic or sulphonic homo- or copolymer (particularly based on AMPS); a film-forming agent, such as a PVP derivative, an acrylic latex or a silicone resin; a dispersant such as a fatty acid ester; an anionic, cationic or non-ionic surfactant and/or emulsifier, particularly a W/O, O/W or W/Si emulsifier; an active substance; an organic or inorganic photoprotective agent or UV filter; a filler; an optionally treated hydrophobic and/or lipophobic pigment; an organic or inorganic powder with a spherical and/or lamellar structure (such as silica, talc, mica, etc.); natural or synthetic fibres; a colorant; a complexing agent; a pH adjuster; a fragrance; a preservative; and mixtures thereof.

According to a preferred embodiment of the invention, one or a plurality of the above-mentioned constituents may be derived from renewable sources, i.e. obtained from raw materials of vegetable, bacterial or animal origin (preferably vegetable).

Furthermore, preferably, this composition is devoid of cyclic silicone (cyclomethicone), particularly cyclotetrasiloxane and cyclopentasiloxane derivatives.

This composition may take the form of a fluid, gel, cream, paste, foam, compressed or cast compact product or a solid product in stick form. It may consists of a facial or body care or hygiene product, particularly a moisturising, anti-age (anti-wrinkle and/or firming), depigmenting, pro-pigmenting or self-tanning, slimming, deodorant or antiperspirant product, or a UV protection product, a cleaner, makeup remover, massage oil or bath product. Alternatively, it may consist of a hair product, particularly a shampoo, conditioner or styling (particularly straightening) product or a hair colour product. Alternatively again, the cosmetic composition according to the invention may consist of a makeup product for the skin, mucosa and/or appendages, particularly a foundation, lipstick, lip gloss, blusher or eye shadow, mascara, eye liner or nail varnish.

The oily composition may particularly be used to improve the wear of mascara, lipstick or foundation and thus produce "transfer-free" compositions. Alternatively, it may be used to improve the evaporation and/or application on the skin of antiperspirant or deodorant compositions, particularly roll-ons or in gel or stick form, particularly based on aluminium salts. Alternatively, it may be used to improve the spreading on skin of sunscreen compositions and/or the dispersion of pigments (especially inorganic nanopigments such as TiO₂ and ZnO) within sunscreen compositions. Therefore, the invention also relates to these uses.

Furthermore, the Applicant found that this oily composition could be used as a vehicle for silicone polymers such as:
- silicone gums, particularly polydimethylsiloxanes hydroxylated at the terminal ends thereof, which are identified under the INCI name DIMETHICONOL,
- emulsifying or non-emulsifying silicone elastomers, which are crosslinked organopolysiloxanes, generally obtained by reacting, in the presence of a catalyst, an organosilicone such as an organohydrogenpolysiloxane with a polysiloxane containing at least one reagent group (hydrogen, allyl or vinyl, in particular) and comprising at least one alkyl (particularly method) or phenyl group and/or at least one polyoxyalkylene group, these groups being situated in a terminal and/or lateral position.

Examples of silicone elastomers are those identified by the INCI name DIMETHICONE / VINYL DIMETHICONE CROSSPOLYMER, particularly available commercially in oily gel form from GRANT INDUSTRIES under the brand Gransil®, from DOW CORNING under the brand name DC 9546® or from GENERAL ELECTRIC under the brand name SFE 839®, for example. Further examples include compounds having the INCI name LAURYL DIMETHICONE / VINYL DIMETHICONE CROSSPOLYMER marketed by SHIN-ETSU, particularly under the brand name KSG-31®. Other silicone elastomers are those having the INCI name DIMETHICONE CROSSPOLYMER, available for example from DOW CORNING under the brand name DC 9040®. Further examples include compounds having the INCI name POLYSILICONE-11, marketed particularly in oily mixture form by GRANT INDUSTRIES under the brand name Gransil® RPS, Gransil® GCM or Gransil® PC-12. A further example includes the compound having the INCI name CROSSLINKED STEARYL METHYL DIMETHYL SILOXANE COPOLYMER available from GRANT INDUSTRIES under the brand name Gransil® SR-CYC.

The oily mixture according to the invention may thus advantageously be used as a substitute for volatile silicone oils or isoparaffins contained in the abovementioned commercial mixtures.

Therefore, the present invention also relates to the use of the oily composition described above as a vehicle for silicone polymers such as elastomers or silicone gums.

The invention will be understood more clearly in the light of the following non-limitative examples, given merely for illustration purposes.

### EXAMPLES

### Example 1: Foundation (W/O emulsion)

Using conventional methods for those skilled in the art, a composition containing the constituents identified in upper case letters in Table 1 below by the INCI name thereof (with reference to the CTFA Dictionary, 11^{th} Edition, 2006), in the percentages by weight specified opposite said constituents, was prepared.

**Table 1**

| Phase | Constituent | % |
|---|---|---|
| A | CETYL PEG/PPG-10/1 DIMETHICONE | 2.80 |
| | PHENYLTRIMETHICONE | 1.00 |
| | POLYDECENE | 3.00 |
| | CETEARYL ETHYLHEXANOATE | 2.00 |
| | Mixture of C₁₂ and C₁₄ linear paraffins | 18.00 |
| B | MICA | 0.50 |
| | RED OXIDE & DIMETHICONE | 0.22 |
| | YELLOW OXIDE & DIMETHICONE | 0.75 |
| | BLACK IRON OXIDE & DIMETHICONE | 0.12 |
| | TITANIUM DIOXIDE & DIMETHICONE | 8.50 |
| C | SODIUM CHLORIDE | 1.25 |
| | PHENOXYETHANOL | 0.50 |
| | BUTYLENE GLYCOL | 5.00 |
| | Water | q.s. 100.00 |
| | Fragrance | q.s. |

### Preparation:

The compounds of phase A were mixed for 10 minutes at 1000 rpm. After the constituents of phase B were mixed, they were added to phase A. The mixture of both phases was homogenised for 30 minutes at 2000 rpm. Phase C was prepared by dispersing the solid constituents thereof in water, and said phase was added to the emulsion previously obtained. The whole was homogenised for 15 minutes at 4500 rpm.

### Evaluation:

The above composition (hereinafter, Composition 1A) was evaluated by a panel of 20 volunteers, compared to an identical formula (hereinafter, Composition 1B), but containing 18.00% by weight of cyclopentasiloxane (DOW CORNING DC 345®) instead of the mixture of C₁₂ and C₁₄ linear paraffins.

These compositions were considered to be identical in terms of the consistency, application homogeneity and covering and transfer-free (on blouse collars) effects thereof. The drying times and matting effect thereof were similar.

Therefore, no significant sensorial differences were perceived between these two formulas.

### Example 2: Dry oil

Using conventional methods for those skilled in the art, a composition 2A containing the constituents identified in Table 2 below (where those in upper case letters are identified by the INCI name thereof), in the percentages by weight specified opposite said constituents, was prepared.

**Table 2**

| Constituent | % |
|---|---|
| CAPRYLIC / CAPRIC TRIGLYCERIDES | 10.00 |
| Jojoba oil | 5.00 |
| SQUALANE | 5.00 |
| OCTYLDODECANOL | 59.90 |
| GLYCINE SOYA & TOCOPHEROL | 0.10 |
| Mixture of C₁₂ and C₁₄ linear paraffins & HEXYL LAURATE | 20.00 |
| Fragrance | q.s. |

This composition was evaluated by a panel of 30 volunteers, compared to the following compositions, in which the oily composition according to the invention was replaced by 20% by weight of the constituents specified below:
Composition 2B: dicaprylyl carbonate (COGNIS Cetiol CC®)
Composition 2C: cyclopentasiloxane
Composition 2D: mixture of isododecane and neopentylglycol diheptanoate (INOLEX Lexfeel® D5)

It was observed that composition 2A according to the invention, like composition 2C, penetrated the skin rapidly, leaving a soft and non-greasy film thereon, whereas composition 2B penetrated more slowly and formed a residual film having a greasy texture. The volatile fraction of composition 2D evaporated rapidly to leave a film with a dry, but grating, unpleasant texture.

The superiority of the oily composition according to the invention was confirmed by substituting hexyl laurate with other esters, i.e.: ethylhexyl isononanoate (composition 2E), isostearyl neopentanoate (composition 2F) and propanediol dicaprylate (composition 2G). The features of all these compositions were equivalent, except that composition 2F gave the skin a more satiny effect.

A similar experiment was conducted in which two compositions 2H and 2H' according to this invention were compared. These were comprised of linear paraffins with 12 and 14 carbon atoms only, in different proportions. Composition 2H contained 30% of C₁₄ paraffin while composition 2H' contained 10% of C₁₄ paraffin. These were compared also with another composition 2H" according to this invention, comprising 1% of C₁₄ paraffin, 9% of an ester oil and 90% of C₁₂ paraffin. Composition 2H provided a softer touch than composition 2H', which was very close to that provided by a standard composition based on Cetiol® CC. Composition 2H" gave similar results to composition 2H' but a gloss somewhat higher which was not detrimental in this application.

### Example 3: Organic anti-age cream

Using conventional methods for those skilled in the art, a composition containing the constituents identified in Table 3 below (where those in upper case letters are identified by the INCI name thereof), in the percentages by weight specified opposite said constituents, was prepared.

**Table 3**

| Constituent | % |
|---|---|
| CETEARYL ALCOHOL & CETEARYL GLUCOSIDE | 5.00 |
| Jojoba oil | 5.00 |
| Camellia oil | 5.00 |
| Shea butter | 5.00 |
| Beeswax | 2.00 |
| Water | q.s. 100.00 |
| Glycerine | 3.00 |
| Xanthan gum | 0.20 |
| Sodium hydroxide | 0.035 |
| Dehydroacetic acid | 0.80 |
| TOCOPHEROL & GLYCINE SOJA | 0.20 |
| Mixture of C₁₂ and C₁₄ linear paraffins & COCO CAPRYLATE/CAPRATE | 1.00 |

This composition was evaluated by a panel of 20 volunteers, compared to identical compositions containing greater quantities of oily composition according to the invention (2, 3 and 5% by weight, respectively).

It was observed that the oily composition according to the invention decreased the soap effect of this cosmetic composition, which had an increasingly soft velvety texture as the oily composition content increased. Besides, this composition provided for a slightly softer touch than DC345® and penetrated quickly into skin.

### Example 4: Anti-perspirant

Using conventional methods for those skilled in the art, a composition containing the constituents identified in Table 4 below (where those in upper case letters are identified by the INCI name thereof), in the percentages by weight specified opposite said constituents, was prepared.

**Table 4**

| Constituent | % |
|---|---|
| CETYL PEG/PPG-10/1 DIMETHICONE | 2.00 |
| TRICLOSAN | 0.10 |
| DIMETHICONOL & VEGETABLE ALKANES (this invention) | 5.00 |
| ALUMINIUM SESQUICHLOROHYDRATE | 15.00 |
| ISODODECANE & DISTEARDIMONIUM HECTORITE & PROPYLENE CARBONATE | 15.00 |
| VEGETABLE ALKANES & COCO CAPRYLATE (this invention) | 56.20 |
| Butylene glycol and spirulina (Spirox®) | 0.03 |
| Fragrance | 6.67 |

### Example 5: Physical properties

The oily mixture of this invention was compared with DC345® and two other paraffin mixtures, i.e. Isopar® M which is a mixture of C₁₃₋₁₄ isoparaffins and Linpar® 14-17 which is a mixture of C₁₄₋₁₇ linear paraffins.

The results of this comparison are summarized in Table 5 below.

**Table 5**

| Component | Flash point (°C)* | Vapour pressure (mm Hg) |
|---|---|---|
| Cyclopentasiloxane and cyclohexasiloxane (DC345®) | 77 | 1 |
| C₁₃-C₁₄ isoparaffin (Isopar® M) | 82 | 0,011 |
| C₁₄₋₁₇ alkanes (Linpar® 14-17) | 118 | < 0,01 |
| Mixture of C₁₂ and C₁₄ alkanes (this invention) | 75 | 0,2 |

| | | |
|---|---|---|
| * according to ASTM D93 | | |

It follows from this Table that this invention provides a mixture of paraffins which has an evaporation profile closer to that of DC345®.

### Example 6: Sensorial analysis

A comparison was made by volunteers between:
- composition 6A: an oily mixture according to this invention consisting of C₁₂ and C₁₄ paraffins and coco caprylate as an ester oil,
- composition 6B: DC345® and
- composition 6C: Lilac® (C₁₄₋₂₂ alkanes).

The following parameters were assessed: smooth effect, quantity of residue, sticky effect, shiny effect, extension, fresh feel, greasy feel and wet effect. The three compositions tested compared similarly, except that composition 6A appeared much less shiny (grade about 5.5) than composition 6C (grade more than 7), with a gloss similar to that of composition 6B (grade about 6).

This example thus demonstrates the superiority of the oily mixture of this invention.

## Claims

1. Volatile oily composition comprising:
(a) from 50 to 100% by weight of a mixture of linear paraffins consisting of:
(i) 70 to 99% by weight of at least one linear paraffin selected from C₈, C₁₀, C₁₂ paraffins and mixtures thereof,
(ii) 1 to 30% by weight of at least one C₁₄ to C₂₄ linear paraffin, and
(b) from 0 to 50% by weight of at least one non-volatile oil selected from: mineral or synthetic branched hydrocarbons, (poly)esters and (poly)ethers C₆-C₂₀ fatty acid triglycerides, vegetable oils, dialkyl carbonates, branched and/or unsaturated fatty acids, branched and/or unsaturated fatty alcohols, silicone oils, fluorosilicone oils, fluorinated oils, and mixtures thereof, wherein the volatile oily composition has a flash point measured according to the ATSM D93 standard below 100 °C, and a vapour pressure from 0.13 Pa (0.001 mm Hg) to 3.99.10⁴ Pa (300 mm Hg) at ambient temperature (20°C) and atmospheric pressure, wherein the non-volatile oil exhibits a vapour pressure less than 0.13 Pa (0.001 mm Hg) at ambient temperature and atmospheric pressure.

2. Composition according to claim 1, **characterised in that** it comprises 90 to 99% by weight, of C₈, C₁₀, C₁₂ paraffin(s) and from 1 to 10% by weight of C₁₄ to C₂₄ paraffin(s).

3. Composition according to claim 2, **characterised in that** it comprises, or consists only of, dodecane and tetradecane.

4. Composition according to any of claims 1 to 3, **characterised in that** it contains 70 to 95% by weight of paraffin mixture and 5 to 30% by weight of non-volatile oil(s).

5. Use of the oily composition according to any of claims 1 to 4 to improve the wear of a mascara, lipstick or foundation, or as a vehicle for silicone polymers, or to improve the spreading on skin of sunscreen compositions and/or the dispersion of pigments within sunscreen compositions.

6. Cosmetic composition containing the oily composition according to any of claims 1 to 4.

7. Composition according to claim 6, **characterised in that** it is devoid of cyclic silicones.

8. Cosmetic use of the cosmetic composition according to any of claims 6 and 7 for making up and/or caring for and/or protecting skin, lips, eyelashes and/or nails.

9. Cosmetic use of the cosmetic composition according to any of claims 6 and 7 to improve the evaporation and/or application on the skin of antiperspirant or deodorant compositions.

## Patentansprüche

1. Flüchtige ölige Zusammensetzung, umfassend:
(a) von 50 bis 100 Gew.-% eines Gemischs aus linearen Paraffinen, bestehend aus:
(i) 70 bis 99 Gew.-% zumindest eines linearen Paraffins, ausgewählt aus C₈, C₁₀, C₁₂-Paraffinen und Gemischen davon,
(ii) 1 bis 30 Gew.-% zumindest eines linearen C₁₄- bis C₂₄-Paraffins und
(b) von 0 bis 50 Gew.-% zumindest eines nicht flüchtigen Öls, ausgewählt aus: mineralischen oder synthetischen verzweigten Kohlenwasserstoffen, (Poly)Estern und (Poly)Ethern, C₆-C₂₀-Fettsäure-Triglyceriden, Pflanzenölen, Dialkylcarbonaten, verzweigten und/oder ungesättigten Fettsäuren. verzweigten und/oder ungesättigten Fettalkoholen, Silikonölen, Fluorsilikonölen, fluorinierten Ölen und Gemischen davon, wobei die flüchtige ölige Zusammensetzung einen gemäß dem Standard ATSM D93 gemessenen Flammpunkt unter 100 °C und einen Dampfdruck von 0,13 Pa (0,001 mmHg) bis 3,99,10⁴ Pa (300 mmHg) bei Umgebungstemperatur (20 °C) und Atmosphärendruck aufweist, wobei das nicht flüchtige Öl einen Dampfdruck von weniger als 0,13 Pa (0,001 mmHg) bei Umgebungstemperatur und Atmosphärendruck aufweist.

2. Zusammensetzung nach Anspruch 1, **dadurch gekennzeichnet, dass** sie 90 bis 99 Gew-% C₈-, C₁₀-, C₁₂-Paraffin(n) und von 1 bis 10 Gew-% C₁₄- bis C₂₄-Paraffin(e) umfasst.

3. Zusammensetzung nach Anspruch 2, **dadurch gekennzeichnet, dass** sie Dodecan und Tetradecan umfasst, oder nur daraus besteht.

4. Zusammensetzung nach einem der Ansprüche 1 bis 3, **dadurch gekennzeichnet, dass** sie 70 bis 95 Gew-% Paraffingemisch und 5 bis 30 Gew-% nicht flüchtiges Öl/nicht flüchtige Öle umfasst.

5. Verwendung der öligen Zusammensetzung nach einem der Ansprüche 1 bis 4, um das Tragen von Mascara, Lippenstift oder Foundation zu verbessern, oder als Vehikel für Silikonpolymere, oder um das Verteilen von Sonnenschutzzusammensetzungen auf der Haut und/oder das Lösen von Pigmenten in den Sonnenschutzzusammensetzungen zu verbessern.

6. Kosmetische Zusammensetzung, die die ölige Zusammensetzung nach einem der Ansprüche 1 bis 4 enthält.

7. Zusammensetzung nach Anspruch 6, **dadurch gekennzeichnet, dass** sie frei von zyklischen Silikonen ist.

8. Kosmetische Verwendung der kosmetischen Zusammensetzung nach einem der Ansprüche 6 und 7 für Make-up und/oder Pflege und/oder Schutz der Haut, Lippen, Wimpern und/oder Nägel.

9. Kosmetische Verwendung der kosmetischen Zusammensetzung nach einem der Ansprüche 6 oder 7, um das Verdunsten und/oder Auftragen auf die Haut von Antiperspirant- oder Deodorantzusammensetzungen zu verbessern.

## Revendications

1. Composition huileuse volatile comprenant :
(a) entre 50 et 100 % en poids d'un mélange de paraffines linéaires comprenant :
(i) entre 70 et 99 % en poids d'au moins une paraffine linéaire choisie parmi les paraffines C₈, C₁₀, C₁₂ et des mélanges de celles-ci,
(ii) entre 1 et 30 % en poids d'au moins une paraffine C₁₄ à C₂₄, et
(b) entre 0 et 50 % en poids d'au moins une huile non volatile choisie parmi : des hydrocarbures ramifiés minéraux ou synthétiques, des (poly)esters et des (poly)éthers, des triglycérides d'acide gras C₆-C₂₀, des huiles végétales, des carbonates de dialkyle, des acides gras ramifiés et/ou insaturés, des alcools gras ramifiés et/ou insaturés, des huiles de silicone, des huiles de fluorosilicone et des mélanges de ceux-ci, dans laquelle la composition huileuse volatile a un point éclair mesuré selon la norme ATSM D93 inférieur à 100 °C et une pression de vapeur comprise entre 0,13 Pa (0,001 mm Hg) et 3,99.10⁴ Pa (300 mm Hg) à température ambiante (20 °C) et à pression atmosphérique, dans laquelle l'huile non volatile présente une pression de vapeur inférieure à 0,13 Pa (0,001 mm Hg) à température ambiante et à pression atmosphérique.

2. Composition selon la revendication 1, **caractérisée en ce qu'**elle comprend entre 90 et 99 % en poids de paraffines C₈, C₁₀, C₁₂ et entre 1 et 10 % en poids de paraffines C₁₄ à C₂₄.

3. Composition selon la revendication 2, **caractérisée en ce qu'**elle comprend uniquement l'un du dodécane et du tétradécane.

4. Composition selon l'une quelconque des revendications 1 à 3, **caractérisée en ce qu'**elle contient entre 70 et 95 % en poids de mélange de paraffine et entre 5 et 30 % en poids d'huiles non volatiles.

5. Utilisation de la composition huileuse selon l'une quelconque des revendications 1 à 4 pour améliorer le port d'un mascara, d'un rouge à lèvres ou d'un fond de teint, ou en tant que véhicule pour des polymères de silicone, ou pour améliorer l'étalement sur la peau de compositions de protection solaire et/ou la dispersion de pigments à l'intérieur de compositions de protection solaire.

6. Composition cosmétique contenant la composition huileuse selon l'une quelconque des revendications 1 à 4.

7. Composition selon la revendication 6, **caractérisée en ce qu'**elle est dépourvue de silicones cycliques.

8. Utilisation cosmétique de la composition cosmétique selon l'une quelconque des revendications 6 et 7 pour maquiller et/ou prendre soin et/ou protéger la peau, les lèvres, les cils et les ongles.

9. Utilisation cosmétique de la composition cosmétique selon l'une quelconque des revendications 6 et 7 pour améliorer l'évaporation et/ou l'application sur la peau de compositions antitranspirantes ou déodorantes.
